Europäisches Patentamt

(19) European Patent Office

Office européen des brevets

(11) Publication number : **0 398 739 B1**

## (12) EUROPEAN PATENT SPECIFICATION

(45) Date of publication of patent specification :
**08.12.93 Bulletin 93/49**

(21) Application number : **90305383.3**

(22) Date of filing : **18.05.90**

(51) Int. Cl.⁵ : **G01N 33/543,** G01N 33/53,
B01L 3/02, B01L 3/14

(54) Filter applicator.

(30) Priority : **18.05.89 US 353841**

(43) Date of publication of application :
**22.11.90 Bulletin 90/47**

(45) Publication of the grant of the patent :
**08.12.93 Bulletin 93/49**

(84) Designated Contracting States :
**DE ES FR GB IT**

(56) References cited :
**EP-A- 0 276 152**
**EP-A- 0 312 394**
**EP-A- 0 321 145**
**WO-A-86/00704**
**GB-A- 1 341 152**
**US-A- 3 985 032**

(73) Proprietor : **QUIDEL CORPORATION**
**10165 McKellar Court**
**San Diego, California 92121 (US)**

(72) Inventor : **Rosman, Daniel B.**
**231 Charter Oak Circle**
**Walnut Creek, CA 94596 (US)**
Inventor : **Rundle, Douglas D.**
**1231 Woodland Avenue**
**Menlo Park, CA 94025 (US)**
Inventor : **Ascani, Gary A.**
**415 Tiller Lane**
**Redwood Shores, CA 94065 (US)**
Inventor : **Mortensen, Richard B.**
**815 - 17th Avenue**
**Menlo Park, CA 94025 (US)**
Inventor : **Tom, Henry**
**P.O.Box 462**
**La Honda, CA 94042 (US)**

(74) Representative : **Harrison, David Christopher**
**et al**
**MEWBURN ELLIS 2 Cursitor Street**
**London EC4A 1BQ (GB)**

Jouve, 18, rue Saint-Denis, 75001 PARIS

## Description

BACKGROUND OF THE INVENTION

The present invention relates generally to methods and apparatus for determining analytes in biological specimens. More particularly, it relates to the construction and use of a liquid sample applicator having an internal matrix which removes particulates from the sample and has a reagent dispersed therein.

The immunoassay of biological specimens has proved to be of enormous benefit in a variety of circumstances, such as the diagnosis of disease, detection of drugs, and monitoring of physiological metabolites. In performing immunoassays on liquid biological samples, pretreatment is frequently required to remove particulates, such as cellular debris, which might foul the assay apparatus or interfere with the assay results. The particulates may be removed, for example, by filtering the liquid sample during a separate step in the assay procedure. Although effective to overcome the interference problems, the addition of a filtering step is undesirable as it adds to the time and complexity of the assay procedure. It would therefore be desirable to provide filtering procedures and apparatus which would be integral to the assay protocol and would not add time or complexity to the overall procedure.

An approach for sample filtration is found in the TestPack® assay system available from Abbott Laboratories, North Chicago, Illinois, and the Preview Serum/Urine-hCG assay system available from Leeco Diagnostics, Inc., Southfield, Michigan. In both cases, a detachable filter is snapped into place against a membrane having bound antibodies specific for the analyte of interest. Sample is applied to the membrane through the filter, and the filter removed prior to development of the assay. Although functional in removing particulates, use of such assay systems has certain disadvantages. In particular, the time required for the sample to penetrate the filter can be as long as one minute or more, adding to the time necessary to perform the assay. Moreover, the need to remove the filter holder prior to development is an extra step which further extends the time required to perform the assay and which, if forgotten, can ruin the test results completely.

Additionally, biological assays often require the combination of one or more reagents with a test sample or other liquid assay component during the course of the assay. Heretofore, reagent addition has generally required discrete addition step(s) performed at appropriate times during the assay. While certainly workable, each additional assay step increases the assay complexity, the time required to perform the assays, and the chance that contaminants and errors will be introduced into the final result. It would therefore be desirable to perform reagent combination(s) concurrently with the transfer of liquid sample or other liquid assay component, thus reducing the number of actual assay steps involved, with a concomitant reduction in time and effort required to perform the assay.

The present invention provides a novel applicator for introducing a reagent to a liquid biological sample or other liquid assay component. The applicator comprises a tube defining an internal lumen and including an integral permeable matrix having a reagent dispersed therein. As a liquid sample or assay component is drawn through the permeable matrix and into the applicator, the reagent component is combined with and released into the sample or other liquid assay component. The sample and reagent are thereafter discharged back through the permeable matrix to an assay medium or device. This results in a reduction in the number of steps in the assay since the requirement of measuring out and adding (as by counting drops) of reagent to the sample or other assay component or of sample and/or reagent to the assay medium or device prior to or following transfer of the sample or other assay component is eliminated. Furthermore, the matrix integrally filters the liquid; surprisingly, the discharge of the sample and reagent back through the matrix is not found to cause any substantial release of particles into the discharging liquid.

In the drawings:

Fig. 1 illustrates a first embodiment of the applicator of the present invention intended for the manual transfer of liquid samples.

Fig. 2 is a schematic illustration of the use of the applicator of the present invention in an automated immunoassay device.

Fig. 3 illustrates another embodiment of the applicator of the present invention which includes a membrane at a fill line in the applicator tube.

Fig. 4 illustrates a further embodiment of the applicator of the present invention which is a one-piece applicator where the vacuum bulb is an integral part of the applicator and is not removeable.

DESCRIPTION OF THE SPECIFIC EMBODIMENTS

According to the present invention, a liquid sample applicator comprises a tube defining an internal lumen and having a filter matrix disposed within the lumen at one end of the tube. The applicator may further include a vacuum bulb at its other end so that it may be used in a manner similar to the use of a conventional fluid dropper in transferring liquid samples in performing immunoassays. Alternatively, the other end of the applicator may be adapted for mounting on a vacuum apparatus which forms part of an automated assay machine. In that case, the applicator may further include immunological reagents immobilized on its internal surface, and the applicator may be used in otherwise conventional automated assay protocols. In both cases, the inclusion of the filter matrix provides bi-directional filtering of the sample fluid to remove particulates which might otherwise interfere in the assay. Such filtering is achieved without additional assay steps or time required for performance of the assay. Surprisingly, it has been found that discharge of the sample through the filter matrix does not result in any appreciable release of the particulates back into the liquid sample.

The present invention is useful in assaying for a wide variety of soluble analytes in virtually any type of biological sample which is liquid or which can be liquified. The method and apparatus will find its greatest use with specimens, such as blood, serum, plasma, urine, cerebral fluid, spinal fluid, ocular lens liquid (tears), saliva, sputum, semen, cervical mucus, scrapings, swab samples, and the like. Use of the filter applicator of the present invention has proved to be particularly valuable in performing urine assays for human chorionic gonadotropin (hCG) and luteinizing hormone (LH) where interference from various particulate and soluble substances has heretofore been problematic.

The samples will generally include particulate contaminants which can interfere in the assay procedure in some manner. For example, the particulates might chemically interfere with the immunological reactions necessary for analyte detection, might mechanically interfere with the assay procedure, such as by plugging ports or membranes, or might interfere with the determination of assay results, such as by affecting optical density readings. Such interfering particulates may derive from a variety of sources, such as cellular debris, mucus, precipitated biological salts, and the like.

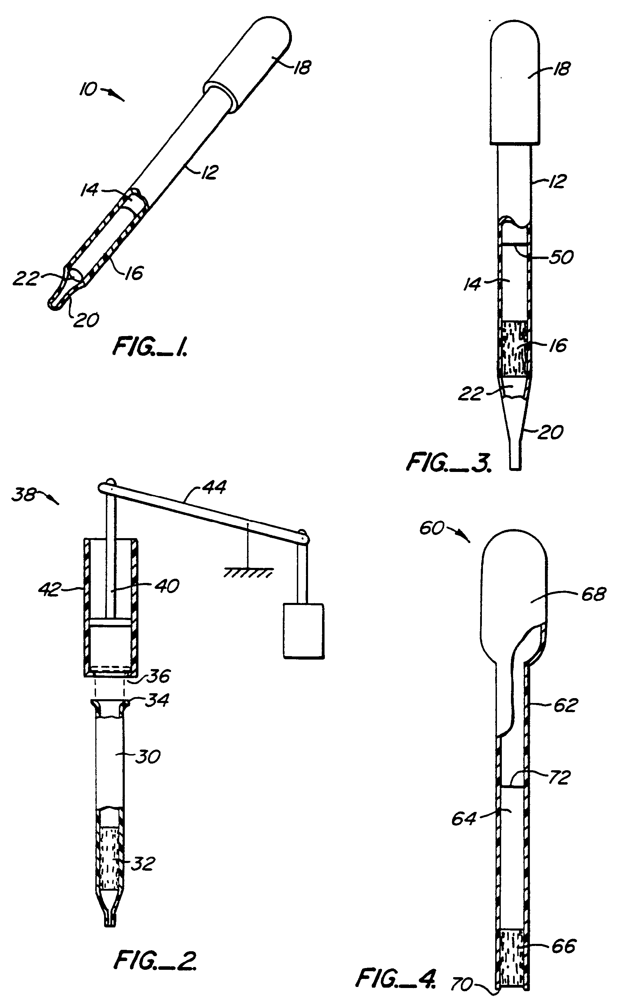

Referring now to Fig. 1, an applicator 10 constructed in accordance with the principles of the present invention includes an elongate tube 12 defining an internal lumen 14 and having a filter matrix 16 disposed within the lumen at one end of the tube. In the first embodiment of Fig. 1, the applicator 10 further includes a vacuum bulb 18 mounted at the end opposite the filter matrix. The bulb 18 is in sealing engagement with the tube 12 so that manipulation of the bulb will cause fluid to be drawn into or expelled from the lumen 14. The tube 11 may be formed from a variety of materials, including glass, metals, ceramics, organic polymers, such as polypropylene, polyvinylchloride, nylon, polyethylene, and the like. The vacuum bulb 15, will typically be formed from an elastomeric polymer, such as natural rubber. Normally, the end 20 of the tube 11 proximate the filter matrix 16 will be tapered. However, this is not required, as is illustrated by end 70 in Fig. 4. Construction of the tube 12 and the vacuum bulb 18 will be similar to that employed for conventional fluid droppers, and commercially available fluid droppers may be used in fabricating the applicators 10 of the present invention.

The filter matrix 16 will be capable of removing particulates having dimensions as small as about 2 $\mu$m, usually as small as about 1 $\mu$m, and preferably as small as about 0.5 $\mu$m and below. The particles, of course, may be much larger, frequently being 3 $\mu$m or greater. At least about 75% of the smallest particulates initially present in the liquid sample will be removed, usually being at least about 90% of such particulates, and more usually being at least about 95% of such particulates. The filters should be formed from a material which is substantially free from binding of the analyte of interest. Frequently, however, it will be desirable for the filter media to remove soluble substances (other than the analyte) including ions, organic molecules, such as creatinines, bilirubins, pigments, and hemoglobin, and inorganic molecules. Suitable materials include polyesters, cellulose acetates, polyacrylonitriles. Particularly preferred are filter matrices formed from poly(ethylene terephthalate) which is a polyester having the empirical formula $[C_{10}H_8O_4]_n$.

In order to obtain efficient particulate removal, it is necessary that the filter matrix 16 be arranged so that the liquid sample will pass through the material rather than merely by the filter material. Thus, certain filter configurations, such as a spiral roll where sample can channel through adjacent layers, are unsuitable. Suitable configurations include placing a filter membrane across the lumen 14 of the applicator 10 so that flow must pass through the membrane, or packing a filter media within the lumen sufficiently tightly so that flow through the media is assured.

The filter matrix 16 is preferably formed from polyester fibers having a diameter in the range from about 5 to 50 $\mu$m, usually in the range from about 10 to 20 $\mu$m. The fibers will be arranged in a bundle having a plurality of fibers arranged parallel to one another at a sufficient density of about $1 \times 10^5$ to $5 \times 10^5$ fibers/cm$^2$, usually being about $2.5 \times 10^5$ fibers/cm$^2$ for fibers having a diameter of 15 $\mu$m. The bundle will usually be mechanically bound, typically in a thin plastic tube, to facilitate handling and insertion into the elongate tube 12. The individual fibers should be free from adhesives and other bonding agents, as adhesives will frequently cause protein binding which will affect the composition of the sample, i.e., will remove analyte therefrom. The length of the fibers will depend on the dimensions of the applicator and the volume of sample to be treated, usually varying in the range from about 0.25 to 5 cm, more usually in the range from about 1.5 to 2.5 cm, and the fiber bundle will be arranged within the tube so that the fibers lie in the axial direction.

Conveniently, the fiber bundle will be inserted through the open end of tube 12 (which is secured by bulb 18) and pushed forward to the location illustrated in Fig. 1. With applicators having tapered tips 20, small void volume 22 will be left within the tip which will hold non-filtered sample as the assay is being performed. As described in more detail hereinafter, it may be desirable to discard a portion of the sample equal to the volume held in the void volume 22 to provide maximum reduction of the particulates in the sample transferred to the assay device or media.

An alternative applicator construction is illustrated in Fig. 2. Applicator tube 30 is constructed similarly to applicator tube 12 and includes a filter matrix 32. Applicator tube 30, however, does not include a vacuum bulb, and instead terminates with an open flange 34 on the open end opposite the filter matrix 32. The flange 34 may be inserted into a port 36 on a vacuum apparatus 38 which forms part of an automated assay machine. The vacuum apparatus 38 is schematically illustrated to include a syringe plunger 40 mounted in a syringe cylinder 42 with a powered lever 44 for reciprocating the plunger 40 up and down. It will be appreciated that when the tube 30 is in place within port 36, as illustrated in broken line, the syringe plunger will be able to draw fluid up into the tube 30 and expel fluid from the tube in a conventional manner.

The construction of the automated assay system may, of course, vary widely, and it is anticipated that the open end of the tube 30 may be adapted to mate with a wide variety of vacuum apparatus in different automated assay machines. Exemplary automated assay machines are disclosed in U.S. Patent Nos. 4,087,248 and 4,447,578. A number of other compatible assay systems are available commercially.

When incorporated in an automated assay system, it may be desirable to coat the inside surface of the applicator tube 30, particularly the portion located above the filter matrix 32 with an immunologically active substance, such as antibody, antigen, and the like. The immobilization of such substances within is amply described in the patent and scientific literature. In this way, certain immunological binding step(s) of the assay may be performed concurrently with the sample transfer step(s).

Another alternative applicator construction is illustrated in Fig. 4. Applicator 60 is constructed of one piece, with the vacuum bulb 68 and tube 62 integrally forming the applicator. Bulb 68 is not removeable from tube 62. Tube 62 defines an internal lumen 64 and has a filter matrix 66 disposed within the lumen at one end of the tube opposite the vacuum bulb. The end 70 proximate the filter matrix 66 is normally flat. A fill line 72 may optionally be included in this or other applicators of the present invention to assist in the determination of volume of sample or other assay component to be drawn up into the applicator in the practice of the invention.

When the fiber bundle comprising the filter matrix 66 is inserted into the one-piece applicator 60, it will conveniently be inserted through the open end 70.

The applicators of the present invention may be used in any assay protocol which employs the use of a fluid dropper or which may be performed in an automated assay machine utilizing detachable pipette tips for sample transfer, incubation, reading, or the like. For example, the applicator 10 may be used for transferring liquid sample from a sample reservoir, such as a specimen cup, to an assay medium or device, such as a test tube, microtiter well, assay membrane, or the like. A particular assay protocol employing a membrane assay device wherein the applicator of the present invention may be utilized is described in U.S. Patent No. 4,818,677.

Generally, the assay device will be capable of immunologically binding the analyte to be detected, and the bound analyte will be visualized on the device, typically by reaction with enzyme-labeled antibody specific for the analyte and exposure to enzyme substrate. Enzyme-substrate systems capable of producing a colored reaction product are amply described in the patent and scientific literature.

Biological assays often require the combination of one or more reagents with the test sample or other liquid assay component during the course of the assay. Heretofore, reagent addition has generally required discrete addition step(s) performed at appropriate times during the assay. While certainly workable, each additional assay step increases the assay complexity, the time required to perform the assays, and the chance that contaminants and errors will be introduced to the final result. It would therefore be desirable to perform reagent combination(s) concurrently with the transfer of liquid sample or other liquid assay component, thus reducing the number of actual assay steps involved, with a concomitant reduction in time and effort required to perform the assay.

Therefore, the present invention further provides a novel applicator for introducing a reagent to a liquid biological sample or other liquid assay component. The applicator includes an integral permeable matrix (usually a filter as described above) having a reagent dispersed therein. As a liquid sample or assay component is drawn through the permeable matrix and into the applicator, the reagent component is combined with and released into the sample or other liquid assay component. The sample and reagent are thereafter discharged through the permeable matrix to an assay medium or device, such as a test tube, microtiter well, assay membrane, or the like. Thus, the number of steps in the assay is reduced since the requirement of measuring out and adding (as by counting drops) of reagent to the sample or other assay component or of sample and/or reagent to the assay medium or device prior to or following transfer of the sample is eliminated.

According to the invention, the applicator for use in introducing a reagent component to a liquid sample or other assay component comprises a tube defining an internal lumen, a permeable matrix disposed within the lumen at one end of the tube, and a reagent dispersed within the permeable matrix. The applicator further includes a means for aspirating sample into the tube and expelling sample from the tube. The means may include a vacuum bulb at the other end of the tube so that it may be used in a manner similar to the use of a conventional fluid dropper in transferring liquid samples or other components in performing assays. Alternatively, the other end of the applicator may be adapted for mounting on a vacuum source which forms part of an automated assay apparatus. Generally, the applicator will be of the general construction as embodied in Figs. 1, 2, 3 and 4.

The permeable matrix 16, 32 or 66 will be chosen from those materials which can reversibly entrap the reagent but will not irreversibly bind it, while allowing passage of the liquid component, so that as the liquid component passes through the matrix the reagent is released. The permeable matrix will usually be formed from filtering materials as described above, including cellulose acetates, bonded or unbonded polyesters, polyacrylonitriles, nylon, cotton, and any other man-made or natural fiber or porous material which meets (or can be modified to meet) the above criteria. Additionally, the permeable matrix may be formed from a variety of inorganic materials, including ceramics, glasses, silicas, and the like, which may be formed into a porous substrate, typically by sintering. If it is desired for the permeable matrix to also be capable of removing particulates, the permeable matrix must also have this filtering characteristic, as discussed previously herein. In one preferred embodiment, the matrices will be filtering matrices formed from poly(ethylene terephthalate), which is a polyester having the empirical formula $[C_{10}H_8O_4]_n$. Other preferred materials include cellulose acetates and polyacrylonitriles.

The volume or mass of the permeable matrix 16 or 32 in the tube 12 or 30 will depend on the physical and/or chemical characteristics of the particular matrix material, the dimensions of the applicator, and the particular reagent to be used and its volume, as well as the volume of sample or other liquid component to be treated. Usually, sufficient permeable matrix material will be provided to promote rapid and uniform transfer of the reagent component into the sample or other liquid. The matrix volume, however, should not be so large that it results in unacceptable hold-up of sample or other liquid in the matrix. The height of the permeable matrix in the exemplary tube embodiment will usually vary in the range from about 0.25 to 5 cm, more usually in the range from about 0.5 to 1.5 cm.

The reagent may be any substance which can react or otherwise interact with the analyte, sample liquid, or other liquid assay component. Such reagents may be chosen from, but are not limited to, salts; buffering salts; free monoclonal or polyclonal antibodies; monoclonal or polyclonal antibody conjugates; free proteins; colloidal suspensions; bead structures; antigens; haptens; cofactors; activators; scavengers; inhibitors; stabilizers; surfactants; and labels, such as enzymes, dyes, fluorescers, chemiluminescers, spin labels, radionuclides, biotin, avidin and the like. Such reagents and their use are amply described in the patent and scientific literature. A comprehensive list of some of the possible reagents is presented in U.S. Patent No. 4,391,904. The reagent may comprise one reagent or a mixture of reagents. In a preferred embodiment of the invention, the reagent is an immunologically active substance, such as antibody, antigen, or the like, which is capable of specifically binding the analyte or competitively binding with the analyte.

The reagent component may be introduced into the permeable matrix by any appropriate method. Desirably, the method will result in the substantially uniform dispersion of the reagent throughout the filter matrix. For example, the permeable matrix may be presaturated by adding liquid reagent component to the matrix prior to insertion of the matrix into the applicator tube. Alternatively, the reagent component may be applied to the permeable matrix after the matrix is placed in the tube by inserting a narrow pipette with reagent into tip 20 and using a vacuum or capillary action to draw the reagent from the pipette into the filter matrix. A fourth alternative would be to utilize a vacuum system to draw liquid reagent into the applicator tip (as generally described in U.S. Patent No. 4,087,248) or to utilize positive pressure to force liquid reagent into the tip (as generally described in U.S. Patent No. 4,447,578). It is also possible to introduce solid phase reagents to the matrix material, typically by combining in a liquid slurry, by any of the techniques just described. A further method may be by inserting a hypodermic needle containing the reagent into tip 20 and injecting the reagent into the filter matrix.

The reagent component in the permeable matrix may be present in a variety of forms, including dried (by air or vacuum), lyophilized, in pellet form (preferably a plurality of discrete pellets dispersed uniformly throughout the matrix), in suspension or emulsion, in a gel, or in a stable liquid form. Liquid reagents will typically be absorbed in the matrix, while solids will be physically entrapped or absorbed within the matrix.

The present invention is useful in introducing a wide variety of reagents to virtually any type of biological sample which is liquid or which can be liquified. The reagent is reconstituted by combining with a liquid sample (or other liquid assay component) as the liquid is drawn through the permeable matrix within the applicator. The large surface area within the matrix over which the reagent is preferably dispersed allows for rapid reconstitution of reagent and thorough mixing with sample or other liquid.

The preferred method of the present invention for introducing a reagent to a liquid biological sample or other assay component comprises:

drawing at least a portion of the liquid sample or assay component from a reservoir into the applicator tube lumen through the permeable matrix, whereby the reagent is reconstituted by and mixed with the liquid sample; and

discharging the combined liquid sample and reagent from the lumen of the applicator tube to an assay medium or device through the permeable matrix in a flow direction opposite to that in which the liquid sample was drawn into the applicator tube.

In a more preferred embodiment of the invention, mixing is further enhanced by introducing air in the form of bubbles into the sample/reagent solution in the lumen of the applicator tube. This action causes bubbles to percolate through the sample/reagent solution to improve mixing efficiency and reaction kinetics to allow for a more rapid reaction. This may be accomplished by drawing air through the permeable matrix behind the measured volume of sample. Air may also be introduced by placing a membrane 50 (Fig. 3) across the applicator tube 12 at a fill line, which membrane will pass air but not liquid once it is wetted. The membrane 50 will allow only the desired volume of sample to enter the applicator, but will allow addition of amounts of air to pass once the applicator tip 20 is removed from the sample. The same effect may be achieved with an impermeable barrier having one or more small orifices sized to allow the passage of air but not sample.

Therefore, the method of the present invention may further comprise introducing air in the form of bubbles into the sample/reagent solution in the applicator tube prior to discharging the sample and reagent from the applicator tube to the assay medium or device.

The applicators of the present invention containing a reagent in the permeable matrix may be used in any assay protocol which employs a step of introducing a reagent to a liquid sample or other liquid assay component. Use of the applicator of this invention allows combination of a liquid transfer step with one or more reagent addition steps, reducing the total number of steps required to perform a particular assay protocol. Moreover, reagent addition in the applicator frequently provides enhanced mixing of the reagent which can reduce the time required to perform an assay protocol and/or increase the accuracy of the assay. When used in an assay where the reagent is an immunologically active substance, the immunological binding step of the assay will typically be performed concurrently with the sample transfer step. For example, analyte may be bound with the immunological reagent as the sample is transferred from a sample reservoir medium to an assay medium or device.

Generally, the assay device will be capable of detecting, visually or otherwise, the bound analyte. For example, bound analyte will be visualized on the device, typically by reaction with enzyme-labeled antibody specific for the analyte and exposure to enzyme substrate. Enzyme-substrate systems capable of producing a colored reaction product are amply described in the patent and scientific literature. A particularly preferred assay device is described in U.S. Patent No. 4,818,677.

The following examples are offered by way of illustration.

EXPERIMENTAL

Example 1:

An applicator as illustrated in Fig. 1 was prepared having a length of 7.2 cm and an internal diameter of 0.53 cm. The filter matrix was composed of poly(ethylene terephthalate) fibers having a length of 2 cm and a diameter of about 15 $\mu$m at a density of $2.5 \times 10^5$ fibers/cm$^2$. A conventional fluid dropper bulb was provided on the upper end of the tube.

hCG positive urine was diluted with hCG negative urine to an approximate concentration of about 50 mIU/mL hCG (WHO First IRP Standard). The urine was subjected to a quantitation assay for hCG before and after being drawn and discharged from the applicator. The results are set forth in Table 1.

## TABLE 1

| | hCG Concentration |
|---|---|
| Prior to Filtration | 65.9 mIU/mL |
| After Filtration | 65.5 mIU/mL |
| Protein Loss | -0.6% |

Thus, the filter resulted in no substantial loss of hCG from the sample.

Example 2:

The applicator from Example 1 was used to perform membrane assays in six samples of fresh morning urine free from hCG. The membrane assay was RAMP™ hCG assays available from Monoclonal Antibodies, Inc., Mountain View, California. Comparative assays were run with conventional fluid droppers without a filter matrix. In both cases, the applicator or fluid dropper was used to transfer fresh sample from a collection cup to the membrane. The time necessary for the sample to be absorbed through the membrane was recorded for each sample for both the applicator and the dropper. The results are set forth in Table 2.

## TABLE 2

### Absorption Time (seconds)

| Specimen No. | Applicator[1] | Applicator[2] | Fluid Dropper (without filter) |
|---|---|---|---|
| 1 | 4 | 5 | 12 |
| 2 | 2.5 | 3 | 3 |
| 3 | 3 | 8 | >60 |
| 4 | 3 | 4 | 6 |
| 5 | 2.5 | 6 | 9 |
| 6 | 3 | ND | 7 |

[1] Average of two tests, first drop discharged.

[2] First drop not discharged.

ND: Not done.

As can be seen from the above results, a substantial improvement in the flow characteristics of the urine samples are achieved with the applicator of the present invention.

Example 3:

Two antibodies specific for different and non-overlapping epitopes on the hCG antigen, each antibody conjugated to the enzyme alkaline phosphatase, were mixed together in a diluent (0.05M Tris, 0.1M NaCl, 0.001M MgCl$_2$) and adjusted to pH 7.4 ("hCG conjugate").

An applicator was prepared as in Example 1, except that the filter matrix was composed of fibers having a length of 1 cm. hCG antibody conjugate solution (25 μL) prepared above was added to the filter by inserting a pipette containing the conjugate solution through the tapered tip of the applicator and applying vacuum. hCG positive urine (400 μL) was then drawn from a sample container into the applicator through the filter matrix, and the applicator was removed from the sample container, after which the bulb was released to allow air bubbles to enter the applicator through the filter matrix. The contents of the applicator were incubated in the tube for 30 sec. and were then applied to a membrane consisting of nylon between two pellon layers to which had been immobilized on the top layer an antibody specific for a third non-overlapping epitope on the hCG antigen. The contents were incubated on the membrane for 1 min. A chromogenic material which produces a blue reaction product when exposed to alkaline phosphatase ("wash/substrate"; 6 drops) was added to the pad, the reaction was incubated for 3 min., and then a solution to stop the reaction was added ("stop"; 4 drops). The results showed a distinct, well-colored blue spot on the membrane with no background.

The above procedure was repeated, except that there was no incubation of the contents in the applicator prior to discharge of the contents onto the membrane. The contents were incubated on the membrane for 10 sec. and treatment was then continued as above. The results gave a lighter blue spot and a clean background.

Example 4:

To the filter of an applicator as in Example 3 was added 50 μL of a 1:1 mixture of glycerol:hCG conjugate. The loaded applicator was held at room temperature for 5 days. 250 mIU/mL hCG urine (500 μL) was then drawn up into the applicator, followed by air, and incubated for 30 s. The contents were dispensed onto a membrane as in Example 3, incubated for 10 seconds and treated as in Example 3. A blue spot of good color intensity resulted.

Example 5:

Lyophilized conjugate was prepared as follows: To assist in dissolving and for protection during freezing, a solution of 2 mg/mL protease-free BSA, 10% mannitol was mixed 1:1 with hCG conjugate of Example 3. This solution (70 μL) was added to an applicator as in Example 3, and the applicator was placed in a lyophilization jar and held at -80°C overnight, after which it was lyophilized and stored at room temperature for 3 days.

A second applicator with filter containing glycerol:hCG conjugate mixture (70 μL) was prepared as in Example 4 and stored at room temperature for 5 days.

Approximately 500 μL of 250 mIU/mL hCG urine was drawn into each of the above two applicators, followed by air, after which the solution in the applicator was immediately discharged onto the membrane pad without an incubation period. As soon as each sample had finished absorbing into the pad, wash/substrate was added without prior incubation. The sample was then incubated for 3 min., after which the reaction was stopped.

Both the lyophilized and the glycerol-containing conjugates gave positive results, with the lyophilized conjugate producing a lighter color response.

Example 6:

Following the procedure of Example 3, 25 μL of hCG conjugate was added to a filter in an applicator. Likewise, 50 μL of 1:1 glycerol:hCG conjugate was added to a filter in another applicator.

Following the shortened assay procedure described in Example 5, 250 mIU/mL hCG urine (500 μL) was added to each of the two applicators and processed. Conjugates from both applicators gave positive results.

## Claims

1. A method for introducing a reagent to a liquid assay component, where the method employs an applicator tube comprising at one end thereof an internal permeable matrix having the reagent dispersed within the matrix, said method comprising:

   drawing at least a portion of the liquid assay component from a reservoir into the applicator tube through the permeable matrix, whereby the reagent is reconstituted by and mixed with the liquid assay component;

   discharging the liquid assay component and reagent from the applicator tube to an assay medium or device through the permeable matrix in a flow direction opposite to that in which the liquid assay component was drawn into the applicator tube.

2. A method as in claim 1, further comprising drawing air through the permeable matrix after a predetermined volume of liquid component has been drawn into the tube.

3. A method as in claim 1, wherein the permeable matrix is composed of a fiber material selected from the group consisting of polyester, cellulose acetate, polyacrylonitrile, and poly(ethylene terephthalate).

4. A method as in claim 1, wherein the reagent is in liquid form and absorbed within the matrix.

5. A method as in claim 1, wherein the reagent is in solid form and physically entrapped or absorbed within the matrix.

6. An applicator for introducing a reagent to a liquid assay component, said applicator comprising:
   a tube defining an internal lumen;
   a liquid-permeable matrix located within the lumen substantially at one end of the tube;
   a reagent dispersed within the filter matrix, said reagent being in a form which will combine with the liquid assay component when exposed thereto; and
   means for aspirating the liquid assay component into the tube through the permeable matrix and expelling the liquid from the tube through the permeable matrix in an opposite flow direction, whereby said reagent is combined with said liquid assay component and discharged.

7. An applicator as in claim 6, wherein the permeable matrix is composed of a fiber material selected from the group consisting of polyester, cellulose acetate, polyacrylonitrile, poly(ethylene terephthalate).

8. An applicator as in claim 6, wherein the reagent is in liquid form and absorbed within the matrix.

9. An applicator as in claim 6, wherein the reagent is in solid form and physically entrapped or absorbed within the matrix.

10. A method for detecting an analyte in sample using an assay device capable of immunologically binding said analyte, said method comprising:
    collecting a liquid sample in a reservoir;
    drawing at least a portion of the liquid sample from the reservoir into an applicator tube having an internal permeable matrix at one end thereof, the permeable matrix having an immunologically active reagent dispersed therein, whereby analyte can bind to the reagent in the liquid phase;
    discharging the liquid component having bound analyte-reagent therein from the applicator tube to the assay device through the matrix in a flow direction opposite to that in which the liquid was drawn into the applicator tube; and
    visualizing the bound analyte on the assay device.


**Patentansprüche**

1. Verfahren zum Einbringen eines Reagens in einen flüssigen Assaybestandteil, wobei im Verfahren ein Applikatorrohr eingesetzt wird, das an einem Ende davon eine innere durchlässige Matrix umfaßt, die das Reagens innerhalb der Matrix dispergiert aufweist, wobei das genannte Verfahren umfaßt:
   das Einsaugen zumindest einer Teilmenge des flüssigen Assaybestandteils aus einem Reservoir in das Applikatorrohr durch die durchlässige Matrix hindurch, wodurch das Reagens durch den flüssigen Assaybestandteil rekonstituiert und mit diesem gemischt wird;
   das Abgeben des flüssigen Assaybestandteils und Reagens aus dem Applikatorrohr in ein(e) Assaymedium oder -vorrichtung durch die durchlässige Matrix hindurch in einer Fließrichtung, die jener entgegengesetzt ist, in der der flüssige Assaybestandteil in das Applikatorrohr eingesaugt wurde.

2. Verfahren nach Anspruch 1, das weiters das Saugen von Luft durch die durchlässige Matrix hindurch umfaßt, nachdem ein vorherbestimmtes Volumen an flüssigem Bestandteil in das Rohr eingesaugt worden ist.

3. Verfahren nach Anspruch 1, worin die durchlässige Matrix aus einem Fasermaterial besteht, das aus der Gruppe ausgewählt ist, die aus Polyester, Zelluloseacetat, Polyacrylnitril und poly(Äthylenterephthalat) besteht.

4. Verfahren nach Anspruch 1, worin das Reagens in flüssiger Form vorliegt und innerhalb der Matrix adsorbiert ist.

**5.** Verfahren nach Anspruch 1, worin das Reagens in fester Form vorliegt und innerhalb der Matrix physikalisch eingeschlossen oder adsorbiert ist.

**6.** Applikator zum Einbringen eines Reagens in einen flüssigen Assaybestandteil, wobei der genannte Applikator umfaßt:

ein Rohr, das ein inneres Lumen begrenzt;

eine flüssigkeitsdurchlässige Matrix, die innerhalb des Lumens im wesentlichen an einem Ende des Rohres angeordnet ist;

ein Reagens, das innerhalb der Filtermatrix dispergiert ist, wobei das genannte Reagens in einer Form vorliegt, die mit dem flüssigen Assaybestandteil kombiniert, wenn es diesem ausgesetzt ist; und

Einrichtungen zum Einsaugen des flüssigen Assaybestandteils in das Rohr durch die durchlässige Matrix hindurch und Austreiben der Flüssigkeit aus dem Rohr durch die durchlässige Matrix hindurch in entgegengesetzter Fließrichtung, wodurch das genannte Reagens mit dem genannten flüssigen Assaybestandteil kombiniert und abgegeben wird.

**7.** Applikator nach Anspruch 6, worin die durchlässige Matrix aus einem Fasermaterial besteht, das aus der Gruppe ausgewählt ist, die aus Polyester, Zelluloseacetat, Polyacrylnitril, poly(Äthylenterephthalat) besteht.

**8.** Applikator nach Anspruch 6, worin das Reagens in flüssiger Form vorliegt und innerhalb der Matrix adsorbiert wird.

**9.** Applikator nach Anspruch 6, worin das Reagens in fester Form vorliegt und innerhalb der Matrix physikalisch eingeschlossen oder adsorbiert ist.

**10.** Verfahren zum Bestimmen eines Analyten in Probe unter Verwendung einer Assayvorrichtung, die fähig ist, den genannten Analyten immunologisch zu binden, wobei das genannte Verfahren umfaßt:

das Sammeln einer flüssigen Probe in einem Reservoir;

das Einsaugen von zumindest einer Teilmenge der flüssigen Probe aus dem Reservoir in ein Applikatorrohr, das eine innere durchlässige Matrix an einem Ende davon aufweist, wobei die durchlässige Matrix ein immunologisch aktives Reagens darin dispergiert aufweist, wodurch Analyt sich an das Reagens in der flüssigen Phase binden kann;

das Abgeben des flüssigen Bestandteils, der gebundenes Analyt-Reagens darin aufweist, aus dem Applikatorrohr an die Assayvorrichtung durch die Matrix in einer Fließrichtung, die der entgegengesetzt ist, in der die Flüssigkeit in das Applikatorrohr eingesaugt wurde; und

das Sichtbarmachen des gebundenen Analyten an der Assayvorrichtung.

## Revendications

**1.** Procédé pour introduire un réactif dans un composé test liquide, où le procédé emploie un tube applicateur comprenant à une extrémité de celui-ci une matrice perméable interne ayant le réactif dispersé à l'intérieur de la matrice, ledit procédé comprenant :

le soutirement d'au moins une partie du composé test liquide d'un réservoir dans le tube applicateur au travers de la matrice perméable, ce par quoi le réactif est reconstitué par et mélangé avec le composé test liquide ;

le déchargement du composé test liquide et du réactif du tube applicateur vers un milieu ou dispositif test à travers la membrane perméable dans une direction d'écoulement opposée à celle dans laquelle le composé test liquide a été introduit dans le tube applicateur.

**2.** Procédé selon la revendication 1, comprenant de plus le soutirement d'air à travers la membrane perméable après qu'un volume prédéterminé de composé liquide ait été introduit dans le tube.

**3.** Procédé selon la revendication 1, dans lequel la matrice perméable est composée d'un matériau fibreux choisi dans le groupe comprenant le polyester, l'acétate de cellulose, le polyacrylonitrile, et le polyéthylène téréphthalate.

**4.** Procédé selon la revendication 1, dans lequel le réactif est sous forme liquide et absorbé dans la matrice.

5. Procédé selon la revendication 1, dans lequel le réactif est sous forme solide et pris au piège ou absorbé dans la matrice.

6. Applicateur pour introduire un réactif dans un composant test liquide, ledit applicateur comprenant :
   un tube définissant un lumen interne ;
   une matrice perméable au liquide située à l'intérieur du lumen substantiellement à une extrémité du tube ;
   un réactif dispersé dans la matrice filtre, ledit réactif étant dans une forme qui se combinera avec un composé test liquide lorsque exposé à celui-ci; et
   des moyens pour aspirer le composé test liquide dans le tube à travers la membrane perméable et l'expulsion du liquide du tube à travers la membrane perméable dans une direction d'écoulement opposée, ce par quoi ledit réactif est combiné avec ledit composé test liquide et déchargé.

7. Applicateur selon la revendication 6, dans lequel la matrice perméable est composée d'un matériau fibreux choisi dans le groupe formé par le polyester, l'acétate de cellulose, le polyacrylonitrile, le polyéthylène téréphthalate.

8. Applicateur selon la revendication 6 dans lequel le réactif est sous forme liquide et absorbé dans la matrice.

9. Applicateur selon la revendication 6, dans lequel le réactif est sous forme solide et physiquement pris au piège ou absorbé dans la matrice.

10. Procédé pour détecter un analyte dans un échantillon en utilisant un dispositif test capable de lier immunologiquement ledit analyte, ledit procédé comprenant :
    la collecte d'un échantillon liquide dans un réservoir ;
    le soutirement d'au moins une partie de l'échantillon liquide du réservoir dans un tube applicateur ayant une matrice perméable interne à une extrémité de celui-ci, la matrice perméable ayant un réactif immunologiquement actif dispersé à l'intérieur, ce par quoi l'analyte peut se lier au réactif dans la phase liquide ;
    le déchargement du composé liquide ayant l'analyte lié au réactif dans son sein du tube applicateur dans le dispositif test à travers la matrice, dans une direction d'écoulement opposée à celle à laquelle le liquide a été introduit dans le tube applicateur ; et
    la visualisation de l'analyte lié sur le dispositif test.

FIG._1.

FIG._3.

FIG._2.

FIG._4.